# EUROPÄISCHE PATENTSCHRIFT

(11) **EP 4 184 151 B1**
(45) Veröffentlichungstag und Bekanntmachung des Hinweises auf die Patenterteilung: **03.07.2024**
(21) Anmeldenummer: 21405006.4
(22) Anmeldetag: 19.11.2021
(51) Int. Cl.: G01N 21/94, B08B 13/00, B08B 3/04

(54) **VERFAHREN ZUR VERIFIZIERUNG DES ERGEBNISSES EINES REINIGUNGSPROZESSES VON ZUVOR AUF EINEM TESTAREAL AUFGETRAGENEN SIMULIERTEN CHEMISCHEN UND/ODER MIKROBIOLOGISCHEN VERUNREINIGUNGEN**
METHOD FOR VERIFYING THE RESULT OF A CLEANING PROCESS OF SIMULATED CHEMICAL AND / OR MICROBIOLOGICAL IMPURITIES PREVIOUSLY APPLIED TO A TEST AREA
PROCÉDÉ DE VÉRIFICATION DU RÉSULTAT D'UN PROCESSUS DE NETTOYAGE DES CONTAMINANTS CHIMIQUES ET/OU MICROBIOLOGIQUES SIMULÉS APPLIQUÉS ANTÉRIEUREMENT À UNE ZONE D'ESSAI

(43) Veröffentlichungstag der Anmeldung: 24.05.2023
(73) Patentinhaber: SKAN AG, 4123 Allschwil (CH)
(72) Erfinder: Hommes, Gregor, D-47669 Wachtendonk (DE); Ravasio, Davide, CH-4612 Wangen bei Olten (CH)
(74) Vertreter: Ullrich, Gerhard

(56) Entgegenhaltungen:
- US-A1- 2013 340 541
- JACK MERRIN ET AL: "Printing Multistrain Bacterial Patterns with a Piezoelectric Inkjet Printer", PLOS ONE, [Online] Bd. 2, Nr. 7, 25. Juli 2007 (2007-07-25), Seite e663, XP055583217, DOI: 10.1371/journal.pone.0000663
- SRIMONGKON TITHIMANAN ET AL: "Application of Biomaterials and Inkjet Printing to Develop Bacterial Culture System", ADVANCES IN MATERIALS SCIENCE AND ENGINEERING, [Online] Bd. 2015, 1. Januar 2015 (2015-01-01), Seiten 1-9, XP055916749, US ISSN: 1687-8434, DOI: 10.1155/2015/290790 Gefunden im Internet: URL:http://downloads.hindawi.com/journals/ amse/2015/290790.pdf>
- Kohli Rajiv: "Chapter 3 - Methods for Assessing Surface Cleanliness" In: "Developments in Surface Contamination and Cleaning, Volume 12 - Methods for Assessment and Verification of Cleanliness of Surfaces and Characterization of Surface Contaminants", 1. Januar 2019 (2019-01-01), XP055916751, Seiten 23-105, Gefunden im Internet: URL:https://doi.org/10.1016/B978-0-12-8160 81-7.00003-6> * das ganze Dokument *

## Beschreibung

### Anwendungsgebiet der Erfindung

Die vorliegende Erfindung betrifft ein Verfahren zur Verifizierung des Ergebnisses eines Reinigungsprozesses von zuvor auf einem Testareal aufgetragenen simulierten chemischen und/oder mikrobiologischen Verunreinigungen. Die Erfindung bezieht sich insbesondere auf den Validierungsprozess für automatisierte Waschanlagenfunktionen der Typen "Cleaning in Place" (CIP) und "Washing in Place" (WIP).

### Stand der Technik

Aus der US 3,841,973 ist ein mechanischer Wischapparat zur automatisierten mikrobiologischen Probenentnahme von einer Oberfläche bekannt. Ein motorisch angetriebener Arm trägt einen Tupfer, der in einem definierten Bewegungsmuster und mit definiertem Andruck über die zu prüfende Oberfläche fährt, wobei der dadurch kontaminierte Tupfer anschliessend analysiert wird. Eine ähnliche Vorrichtung zur automatisierten Reinigungsvalidierung für eine zu überprüfende Oberfläche wird in der WO 2014/089 320 A1 beschrieben. Nach dem Reinigungsprozess dennoch auf der Oberfläche verbliebene Schmutzreste werden von einem auf der Oberfläche bewegten Tupfer aufgenommen und mittels Analyse quantifiziert.

Die US 6,789,965 B2 hat eine Vorrichtung und ein Verfahren zum Aufbringen biologischer oder chemischer Substanzen durch Kapillarröhrchen in hochauflösender und definierter Formation auf eine Testplatte zu Diagnosezwecken, zum Beispiel für DNA-Analysen, zum Gegenstand.

Der Flyer "Cleanability of Surfaces - CSM Riboflavin Test" des Fraunhofer-Instituts für Produktionstechnik und Automatisierung IPA [siehe: https://www.ipa.fraunhofer.de/content/dam/ipa/en/documents/Expertises/Reinst-und-Mikroproduktion/Flyer Cleanability_of Surfaces.pdf (fraunhofer.de) Flyer_Cleanability_of Surfaces.pdf (fraunhofer.de)] befasst sich ebenfalls mit der hiesigen Thematik. Beschrieben wird ein Testverfahren zur Überprüfung der Wirksamkeit vollständiger Reinigungs-/Waschzyklen auf Oberflächen mit Verwendung einer Testflüssigkeit, welche das wasserlösliche und sehr lichtempfindliche B2-Vitamin Riboflavin enthält.

Für einen Test werden zunächst die zu reinigenden Oberflächen der betreffenden Apparaturen mit einer Wasser-Riboflavin-Lösung besprüht. Anschliessend besprüht man diese Oberflächen mit einer Spüllösung gemäss der Burst-Spül-Methode im zeitgesteuerten Modus, gefolgt von einer zeitgesteuerten Schwerkraftdrainage, vorzugsweise dreimalig. Nach Abschluss der Spülphase werden insbesondere sehr heikle Oberflächenareale mittels Schwarzlicht und Teleskopspiegel visuell überprüft, um festzustellen, ob die Riboflavin-Lösung vollständig entfernt wurde. Keine sichtbare Fluoreszenz bestätigt die vollständige, ordnungsgemässe Reinigung. Zeigen sich jedoch unter dem Schwarzlicht Rückstände, erkennt der Techniker unzureichend gereinigte Oberflächenareale und muss die Intensität und/oder die Zielrichtung der Sprüheinrichtung anpassen.

Die US 2013/0340541 A1 schliesslich offenbart ein Verfahren zur Verifizierung des Ergebnisses eines Reinigungsprozesses von zuvor auf einem Testareal aufgetragenen simulierten chemischen und/oder mikrobiologischen Verunreinigungen, gemäss dem Oberbegriff des unabhängigen Anspruchs 1, mit der Schrittfolge:
- Bereitstellung eines Beschichtungsgerätes mit gefüllter Kartusche, welche eine Testflüssigkeit zur Simulation chemischer und/oder mikrobiologischer Verunreinigungen beinhaltet, wobei die Testflüssigkeit eine definierte Art und Konzentration an chemisch und/oder mikrobiologisch verunreinigender Substanzen enthält;
- Auswahl eines definierten Testareals und Auftrag einer definierten Menge an Testflüssigkeit auf das Testareal mit dem Beschichtungsgerät;
- Durchführung eines standardisierten Reinigungsprozesses des mit der Testflüssigkeit versehenen Testareals; und
- Verifizierung und Protokollierung des Ergebnisses des durchgeführten Reinigungsprozesses.

Weitere für diese Erfindung relevante Nicht-Patentliteraturdokumente sind die folgenden Dokumente:
- Kohli Rajiv: "Chapter 3 - Methods for Assessing Surface Cleanliness"In: "Developments in Surface Contamination and Cleaning, Volume 12 - Methods for Assessment and Verification of Cleanliness of Surfaces and Characterization of Surface Contaminants", 1. Januar 2019
- Srimongkon Tithimanan et al: "Application of Biomaterials and Inkjet Printing to Develop Bacterial Culture System"
- Jack Merrin et al: "Printing Multistrain Bacterial Patterns with a Piezoelectric Inkjet Printer".

### Aufgabe der Erfindung

Zwar erbringt der Riboflavin-Test eine gewisse qualitative Überprüfung der Gründlichkeit der durchgeführten Reinigung, jedoch keinen quantitativen Nachweis, ob zur Vermeidung von Hygiene- oder Kreuzkontaminationsrisiken die im Reinigungsprozess behandelten Oberflächen tatsächlich völlig frei von hochpotenten oder sensibilisierenden Substanzen sind, beziehungsweise nur mehr mit äusserst niedrigen, das heisst noch akzeptablen Rückständen belastet sind, was für eine qualifizierte Validierung unerlässlich ist.

Die Verantwortung für die Validierung vorschriftsgemässer Reinigung (Performance Qualification - PQ) von insbesondere in der Pharmaindustrie zu behandelnden Apparaturoberflächen und eingesetzten Funktionsteilen, mittels Waschvorrichtungen, liegt erst letztendlich bei den Anwendern, jedoch erwarten die Anwender der Waschvorrichtungen von den Herstellern auch die Bereitstellung von Validierungsverfahren zum Nachweis anspruchsvollsten Reinigungsgrades. Der vorliegenden Erfindung liegt daher die Aufgabe zugrunde, für das betreffende Anwendungsgebiet ein in seiner Anwendung effizientes und höchst zuverlässiges und sensibles Validierungsverfahren vorzuschlagen.

### Übersicht über die Erfindung

Das erfindungsgemässe Verfahren ist zur Verifizierung des Ergebnisses eines Reinigungsprozesses von zuvor auf einem Testareal aufgetragenen simulierten chemischen und/oder mikrobiologischen Verunreinigungen konzipiert und umfasst die Schritte gemäß Anspruch 1.

Nach dem Auftrag der definierten Menge an Testflüssigkeit auf das Testareal und vor der Durchführung des standardisierten Reinigungsprozesses des mit der Testflüssigkeit versehenen Testareals wird die Intensität und gegebenenfalls auch die Art der auf dem Testareal vorhandenen Verunreinigung ermittelt und protokolliert. Das Testareal wird auf der Oberfläche einer Anlage bestimmt, zum Beispiel einem Containment zu Produktions- oder Forschungszwecken pharmazeutischer oder biotechnischer Erzeugnisse oder in der Medizintechnik. Das Testareal lässt sich auch auf der Oberfläche eines in der Anlage einsetzbaren Funktionsteils bestimmen.

Alternativ dient als Testareal ein separates Materialstück in Gestalt eines Indikatorplättchens, welches in seiner stofflichen Charakteristik zu dem im Anlagenbau beziehungsweise zu dem für das Funktionsteil verwendeten Material identisch ist, jedenfalls als Referenzmaterial akzeptabel ist und zum Beispiel auch als Folie beschaffen sein kann.

Das für die zur Simulation chemischer und/oder mikrobiologischer Verunreinigungen und mit der Testflüssigkeit gefüllten Kartusche bereitzustellende Beschichtungsgerät ist vom Typ eines Tintenstrahldruckers. In vervollkommneter Ausbildung ist das Beschichtungsgerät auf verschiedene Beschichtungsmengen sowie Beschichtungsmuster und/oder auf verschieden dimensionierte Testareale einstellbar und hat vorzugsweise die Gestalt eines Handgeräts.

Die der Testflüssigkeit zuzusetzende verunreinigende Substanz sind Partikel und/oder ein Solvat. Die Testflüssigkeit besteht hauptsächlich aus einem neutralen Solvens und diesem zugesetzten chemischen und/oder mikrobiologischen Substanzen, wie Viren, Pilze, Sporen, Bakterien, Proteinen oder Zellfragmenten, in definierter Konzentration zur Simulation von Verunreinigungen auf dem Testareal.

Alternativ besteht die Testflüssigkeit wiederum hauptsächlich aus einem neutralen Solvens und im Solvens enthaltenen chemischen Substanzen und/oder einem Zwei- oder Mehrkomponenten-Gemisch mikrobiologischer Substanzen aus der Gruppe, Viren, Pilze, Sporen, Bakterien, Proteine und Zellfragmente, in definierter Konzentration zur Simulation von Verunreinigungen auf dem Testareal.

Die Verifizierung des Ergebnisses des durchgeführten Reinigungsprozesses bei Verwendung eines Testareals erster Ausprägung umfasst folgende Schritte:
- Überwischen der zuvor kontaminierten und anschliessend gereinigten Oberfläche des Testareals mit einem neutralen Putzelement;
- Herauslösen der beim Überwischen vom Putzelement von der Oberfläche des Testareals aufgenommenen Spuren mit einem Lösungsmittel; und
- Untersuchen des spuren-belasteten Lösungsmittels in einem Analysator; wobei
- das Analyseergebnis anzeigt, ob der Reinigungsprozess erfolgreich war oder ob Rückstände der verunreinigenden Testflüssigkeit über das Zulässigkeitsmass auf dem Testareal verblieben sind, so dass der Reinigungsprozess wiederholt bzw. von Anbeginn intensiviert werden muss, um einen akzeptablen Reinheitsgrad zu erzielen.

Die Verifizierung des Ergebnisses des durchgeführten Reinigungsprozesses bei Verwendung eines Testareals zweiter Ausprägung umfasst folgende Schritte:
- Behandlung der zuvor kontaminierten und anschliessend gereinigten Oberfläche des Testareals zweiter Ausprägung in einem Gefäss mit einem Lösungsmittel; und
- Untersuchen des spuren-belasteten Lösungsmittels in einem Analysator; wobei
- das Analyseergebnis anzeigt, ob der Reinigungsprozess erfolgreich war oder ob Rückstände der verunreinigenden Testflüssigkeit über das Zulässigkeitsmass auf dem Testareal verblieben sind, so dass der Reinigungsprozess wiederholt beziehungsweise von Anbeginn intensiviert werden muss, um einen akzeptablen Reinheitsgrad zu erzielen.

Vor dem Auftrag der Testflüssigkeit auf das als Testareal gewählte Indikatorplättchen mittels des Beschichtungsgeräts dient eine Positioniervorrichtung zur exakten Ausrichtung des Indikatorplättchens unter dem Druckerkopf des Beschichtungsgeräts.

### Ausführunqsbeispiel

Das zur Verifizierung des Ergebnisses eines insbesondere von einer Waschvorrichtung ausgeführten Reinigungsprozesses von zuvor auf einem Testareal aufgetragenen simulierten chemischen und/oder mikrobiologischen Verunreinigungen geschaffene Verfahren umfasst die Abfolge nachstehender Schritte:

### Schritt 1

Bereitstellung eines Beschichtungsgeräts mit gefüllter Kartusche, welche eine Testflüssigkeit zur Simulation chemischer und/oder mikrobiologischer Verunreinigungen beinhaltet, wobei die Testflüssigkeit eine definierte Art und Konzentration an chemisch und/oder mikrobiologisch verunreinigenden Substanzen enthält. Die der Testflüssigkeit zuzusetzende verunreinigende Substanz sind Partikel und/oder ein Solvat. Die Testflüssigkeit besteht hauptsächlich aus einem neutralen Solvens und diesem zugesetzten chemischen und/oder mikrobiologischen Substanzen, wie Viren, Pilze, Sporen, Bakterien, Proteinen oder Zellfragmenten, in definierter Konzentration zur Simulation von Verunreinigungen auf dem Testareal. Alternativ besteht die Testflüssigkeit wiederum hauptsächlich aus einem neutralen Solvens und im Solvens enthaltenen chemischen Substanzen und/oder einem Zwei- oder Mehrkomponenten-Gemisch mikrobiologischer Substanzen aus der Gruppe, Viren, Pilze, Sporen, Bakterien, Proteine und Zellfragmente, in definierter Konzentration zur Simulation von Verunreinigungen auf dem Testareal.

Das für die zur Simulation chemischer und/oder mikrobiologischer Verunreinigungen und mit der Testflüssigkeit gefüllten Kartusche bereitzustellende Beschichtungsgerät ist vom Typ eines Tintenstrahldruckers. In vervollkommneter Ausbildung ist das Beschichtungsgerät auf verschiedene Beschichtungsmengen sowie Beschichtungsmuster und/oder auf verschieden dimensionierte Testareale einstellbar und hat vorzugsweise die Gestalt eines Handgeräts.

### Schritt 2

Auswahl eines definierten Testareals und Auftrag einer definierten Menge an Testflüssigkeit auf das Testareal. Das Testareal wird auf der Oberfläche einer Anlage bestimmt, zum Beispiel einem Containment zu Produktions- oder Forschungszwecken pharmazeutischer oder biotechnischer Erzeugnisse oder in der Medizintechnik. Das Testareal kann auch auf der Oberfläche eines in der Anlage einsetzbaren Funktionsteils bestimmt sein. Alternativ dient als Testareal ein separates Materialstück, welches in seiner stofflichen Charakteristik zu dem im Anlagenbau beziehungsweise zu dem für das Funktionsteil verwendeten Material identisch ist, das jedenfalls als Referenzmaterial akzeptabel ist. Das Testareal kann somit in zwei verschiedenen Ausprägungen bestimmt werden. Die erste Ausprägung ist ein reeller Oberflächenabschnitt der Anlage oder eines darin einsetzbaren Funktionsteils. Hingegen ist die zweite Ausprägung ein Indikatorplättchen, entweder bestehend aus einem zum reellen Oberflächenabschnitt identischen einzelnen Materialstück oder einem adäquaten Referenzmaterial. Nach dem Auftrag der definierten Menge an Testflüssigkeit auf das Testareal gemäss erster oder zweiter Ausprägung und vor der Durchführung des standardisierten Reinigungsprozesses des mit der Testflüssigkeit versehenen Testareals wird die Intensität und gegebenenfalls auch die Art der auf dem Testareal vorhandenen Verunreinigung ermittelt und protokolliert. Der Auftrag der Testflüssigkeit auf das Testareal erfolgt vorzugsweise durch Besprühen, insbesondere Bedruckung.

### Schritt 3

Durchführung eines standardisierten Reinigungsprozesses des mit der Testflüssigkeit versehenen Testareals gemäss erster oder zweiter Ausprägung. Ein solcher Reinigungsprozess erfolgt durch Besprühen mittels einer Spüllösung, vorzugsweise in zeit- und in der Intensität gesteuertem Modus und erforderlichenfalls in mehreren Zyklen. Die Abfuhr der Spüllösung geschieht, bei entsprechender Eignung, durch Schwerkraftdrainage oder durch Absaugen.

### Schritt 4

Nach den vorausgegangenen Schritten erfolgt die Verifizierung und Protokollierung des Ergebnisses des durchgeführten Reinigungsprozesses. Der gesamte Verfahrensablauf lässt sich nun wie folgt zusammenfassen, wobei sich eine Differenzierung danach ergibt, ob das Testareal in seiner ersten oder zweiten Ausprägung gewählt wird. Also wird gemäss Schritt 2 ein reeller Oberflächenabschnitt der Anlage oder eines darin einsetzbaren Funktionsteils als Testareal erster Ausprägung oder ein Indikatorplättchen als Testareal zweiter Ausprägung bestimmt.

### Verfahrensablauf mit Testareal erster Ausprägung (reeller Oberlächenabschnitt)

Mit dem Beschichtungsgerät gemäss Schritt 1 wird auf das Testareal eine definierte Menge an Testflüssigkeit aufgetragen. Als nächstes erfolgt gemäss Schritt 3 die Durchführung eines standardisierten Reinigungsprozesses des mit der Testflüssigkeit versehenen Testareals.

Schliesslich steht Schritt 4 mit der Überprüfung an, ob das Resultat des durchgeführten Reinigungsprozesses als vorschriftsgemäss akzeptabel ist. Hierzu wird die zuerst kontaminierte und anschliessend im Schritt 3 gereinigte Oberfläche des Testareals mit einem neutralen Putzelement übergewischt. Anschliessend werden die beim Überwischen vom Putzelement von der Oberfläche des Testareals aufgenommenen Spuren mit einem Lösungsmittel herausgelöst und in einem Analysator untersucht. Das Analyseergebnis zeigt an, ob der Reinigungsprozess erfolgreich war oder ob Rückstände der verunreinigenden Testflüssigkeit über das Zulässigkeitsmass auf dem Testareal verblieben sind, so dass der Reinigungsprozess wiederholt beziehungsweise von Anbeginn intensiviert werden muss, um einen akzeptablen Reinheitsgrad zu erzielen.

### Verfahrensablauf mit Testareal zweiter Ausprägung (Indikatorplättchen)

Nach sich bewährender Praxis wird als Testareal ein Indikatorplättchen verwendet - "Coupon" in der Fachbezeichnung -, welches der vorstehenden Charakterisierung entspricht. Vor dem Auftragen der Testflüssigkeit auf das Indikatorplättchen mit dem Beschichtungsgerät gemäss Schritt 1 setzt man das Indikatorplättchen zu dessen exakter Ausrichtung unter dem Druckerkopf des Beschichtungsgeräts in eine Positioniervorrichtung ein. Wiederum wird nun eine definierte Menge an Testflüssigkeit aufgetragen und anschliessend, gemäss Schritt 3, erfolgt die Durchführung eines standardisierten Reinigungsprozesses des mit der Testflüssigkeit versehenen Indikatorplättchens. Vorzugsweise verbleibt dabei das Indikatorplättchen in der Positioniervorrichtung.

Abschliessend ist im Schritt 4 zu überprüfen, ob der durchgeführte Reinigungsprozess ein einwandfreies Resultat erbrachte. Hierzu wird das zuerst gezielt kontaminierte und anschliessend im Schritt 3 gereinigte Indikatorplättchen in einem Gefäss mit Lösungsmittel behandelt. Die dadurch herausgelösten Rückstände werden in einem Analysator untersucht. Aus dem Analyseergebnis resultiert die Erkenntnis, ob der Reinigungsprozess ausreichend wirksam war oder ob von der kontaminierenden Testflüssigkeit auf dem Indikatorplättchen in nicht akzeptablem Mass Rückstände verblieben sind und somit zum Erreichen des verlangten Reinheitsgrads die Reinigung wiederholt bzw. von Anbeginn verstärkt werden muss.

## Patentansprüche

1. Verfahren zur Verifizierung des Ergebnisses eines Reinigungsprozesses von zuvor auf einem Testareal aufgetragenen simulierten chemischen und/oder mikrobiologischen Verunreinigungen, umfassend folgende Schritte:
a) Bereitstellung eines Beschichtungsgerätes mit gefüllter Kartusche, welche eine Testflüssigkeit zur Simulation chemischer und/oder mikrobiologischer Verunreinigungen beinhaltet, wobei die Testflüssigkeit eine definierte Art und Konzentration an chemisch und/oder mikrobiologisch verunreinigender Substanzen enthält;
b) Auswahl eines definierten Testareals und Auftrag einer definierten Menge an Testflüssigkeit auf das Testareal mit dem Beschichtungsgerät;
c) Durchführung eines standardisierten Reinigungsprozesses des mit der Testflüssigkeit versehenen Testareals; und
d) Verifizierung und Protokollierung des Ergebnisses des durchgeführten Reinigungsprozesses, **gekennzeichnet durch**:
e) einen Tintenstrahldrucker als Beschichtungsgerät; und
f) folgende Verifizierungsschritte:
fa) Überwischen der zuvor kontaminierten und anschliessend gereinigten Oberfläche des Testareals in einer ersten Ausprägung mit einem neutralen Putzelement und Herauslösen der beim Überwischen vom Putzelement von der Oberfläche des Testareals aufgenommenen Spuren mit einem Lösungsmittel; oder
fb) Behandlung der zuvor kontaminierten und anschliessend gereinigten Oberfläche des Testareals in einer zweiten Ausprägung in einem Gefäss mit einem Lösungsmittel; und
fc) Untersuchen des spuren-belasteten Lösungsmittels in einem Analysator; wobei
fd) das Analyseergebnis anzeigt, ob der Reinigungsprozess erfolgreich war oder ob Rückstände der verunreinigenden Testflüssigkeit über das Zulässigkeitsmass auf dem Testareal verblieben sind, so dass der Reinigungsprozess wiederholt bzw. von Anbeginn intensiviert werden muss, um einen akzeptablen Reinheitsgrad zu erzielen.

2. Verfahren nach Anspruch 1, **dadurch gekennzeichnet, dass** nach dem Auftrag der definierten Menge an Testflüssigkeit auf das Testareal und vor der Durchführung des standardisierten Reinigungsprozesses des mit der Testflüssigkeit versehenen Testareals die Intensität und möglicherweise auch die Art der auf dem Testareal vorhandenen Verunreinigung ermittelt und protokolliert wird.

3. Verfahren nach zumindest einem der Ansprüche 1 und 2, **dadurch gekennzeichnet, dass** das Testareal in der ersten Ausprägung bestimmt wird auf der Oberfläche:
a) einer Anlage, zum Beispiel einem Containment zu Produktions- oder Forschungszwecken pharmazeutischer oder biotechnischer Erzeugnisse oder in der Medizintechnik; oder
b) eines in der Anlage einsetzbaren Funktionsteils.

4. Verfahren nach zumindest einem der Ansprüche 1 und 2, **dadurch gekennzeichnet, dass** als Testareal in der alternativen zweiten Ausprägung ein separates Materialstück in Gestalt eines Indikatorplättchens dient, welches in seiner stofflichen Charakteristik zu dem im Anlagenbau beziehungsweise zu dem für das Funktionsteil verwendeten Material identisch ist oder jedenfalls als Referenzmaterial akzeptabel ist.

5. Verfahren nach Anspruch 4, **dadurch gekennzeichnet, dass** vor dem Auftrag der Testflüssigkeit auf das als Testareal gewählte Indikatorplättchen mittels des Beschichtungsgeräts eine Positioniervorrichtung zur exakten Ausrichtung des Indikatorplättchens unter dem Druckerkopf des Beschichtungsgeräts dient.

6. Verfahren nach zumindest einem der Ansprüche 1 bis 5, **dadurch gekennzeichnet, dass** das Beschichtungsgerät auf verschiedene Beschichtungsmengen und Beschichtungsmuster einstellbar ist.

7. Verfahren nach zumindest einem der Ansprüche 1 bis 6, dadurch qekennzeichnet, dass das Beschichtungsgerät auf verschieden dimensionierte Testareale einstellbar ist.

8. Verfahren nach zumindest einem der Ansprüche 1 bis 7, **dadurch gekennzeichnet, dass** das Beschichtungsgerät als Handgerät ausgebildet ist.

9. Verfahren nach zumindest einem der Ansprüche 1 bis 8, **dadurch gekennzeichnet, dass** die der Testflüssigkeit zuzusetzende verunreinigende Substanz Partikel und/oder ein Solvat sind.

10. Verfahren nach zumindest einem der Ansprüche 1 bis 9, **dadurch gekennzeichnet, dass** die Testflüssigkeit besteht aus:
a) einem neutralen Solvens; und
b) im Solvens enthaltenen chemischen und/oder mikrobiologischen Substanzen, wie Viren, Pilze, Sporen, Bakterien, Proteinen oder Zellfragmenten, in definierter Konzentration zur Simulation von Verunreinigungen auf dem Testareal.

11. Verfahren nach Anspruch 10, **dadurch gekennzeichnet, dass** die Testflüssigkeit besteht aus:
a) einem neutralen Solvens; und
b) im Solvens enthaltenen chemischen Substanzen und/oder ein Zwei- oder Mehrkomponenten-Gemisch mikrobiologischer Substanzen aus der Gruppe, Viren, Pilze, Sporen, Bakterien, Proteine und Zellfragmente, in definierter Konzentration zur Simulation von Verunreinigungen auf dem Testareal.

## Claims

1. Method for verifying the result of a cleaning process to remove simulated chemical and/or microbiological contaminants previously applied to a test area, comprising the following steps:
a) providing a coating device having a filled cartridge containing a test liquid for simulation of chemical and/or microbiological contaminants, wherein the test liquid contains a defined type and concentration of chemically and/or microbiologically contaminating substances;
b) selecting a defined test area and applying a defined quantity of test liquid to the test area by means of the coating device;
c) performing a standardized cleaning process on the test area provided with the test liquid; and
d) verifying and logging the result of the cleaning process performed, **characterized by**:
e) an inkjet printer as the coating device; and
f) the following verification steps:
fa) wiping over the previously contaminated and subsequently cleaned surface of the test area in a first form with a neutral cleaning element, and removing the traces picked up from the surface of the test area by the cleaning element on wiping, using a solvent; or
fb) treating the previously contaminated and subsequently cleaned surface of the test area in a second form in a vessel containing a solvent; and
fc) testing the traces-loaded solvent in an analyser; wherein
fd) the analysis result indicates whether the cleaning process was successful or whether residues of the contaminating test liquid above the permissible level remained on the test area, meaning that the cleaning process must be repeated or be intensified from the beginning in order to achieve an acceptable level of cleanliness.

2. Method according to Claim 1, **characterized in that**, after the application of the defined quantity of test liquid to the test area and before the performance of the standardized cleaning process on the test area provided with the test liquid, the intensity and possibly also the type of the contaminant present on the test area is determined and logged.

3. Method according to at least one of Claims 1 and 2, **characterized in that** the test area in the first form is defined on the surface:
a) of an installation, for example a containment for production or research purposes in relation to pharmaceutical or biotechnological products or in medical technology; or
b) of a functional part usable in the installation.

4. Method according to at least one of Claims 1 and 2, **characterized in that** the test area used in the alternative second form is a separate piece of material in the form of an indicator plate which is identical in terms of its material characteristics to the material used in the construction of the installation or to the material used for the functional part or which is acceptable as reference material in any case.

5. Method according to Claim 4, **characterized in that**, before the application of the test liquid to the indicator plate selected as test area by means of the coating device, a positioning device is used for exact alignment of the indicator plate under the printer head of the coating device.

6. Method according to at least one of Claims 1 to 5, **characterized in that** the coating device is adjustable to different coating quantities and coating patterns.

7. Method according to at least one of Claims 1 to 6, **characterized in that** the coating device is adjustable to test areas of different dimensions.

8. Method according to at least one of Claims 1 to 7, **characterized in that** the coating device is designed as a handheld device.

9. Method according to at least one of Claims 1 to 8, **characterized in that** the contaminating substance to be added to the test liquid are particles and/or a solvate.

10. Method according to at least one of Claims 1 to 9, **characterized in that** the test liquid consists of:
a) a neutral solvent; and
b) chemical and/or microbiological substances present in the solvent, such as viruses, fungi, spores, bacteria, proteins or cellular fragments, in a defined concentration for simulation of contaminants on the test area.

11. Method according to Claim 10, **characterized in that** the test liquid consists of:
a) a neutral solvent; and
b) chemical substances present in the solvent and/or a two-component or multicomponent mixture of microbiological substances from the group consisting of viruses, fungi, spores, bacteria, proteins and cellular fragments, in a defined concentration for simulation of contaminants on the test area.

## Revendications

1. Procédé de vérification du résultat d'un procédé de nettoyage d'impuretés chimiques et/ou microbiologiques simulées appliquées au préalable sur une zone de test, comprenant les étapes suivantes:
a) mise à disposition d'un appareil de revêtement, pourvu de cartouches remplies, qui comporte un liquide de test pour la simulation d'impuretés chimiques et/ou microbiologiques, le liquide de test contenant un type défini et une concentration définie de substances de contamination chimique et/ou microbiologique;
b) sélection d'une zone de test définie et application d'une quantité définie de liquide de test sur la zone de test à l'aide de l'appareil de revêtement;
c) réalisation d'un procédé de nettoyage standardisé de la zone de test pourvue du liquide de test; et
d) vérification et consignation du résultat du procédé de nettoyage réalisé, **caractérisé par**:
e) une imprimante à jet d'encre comme appareil de revêtement; et
f) les étapes de vérification suivantes:
fa) essuyage de la surface contaminée au préalable et ensuite nettoyée de la zone de test, dans une première forme, à l'aide d'un élément de nettoyage neutre et dissolution des traces reprises lors de l'essuyage de l'élément de nettoyage de la surface de la zone de test par un solvant; ou
fb) traitement de la surface contaminée au préalable et ensuite nettoyée de la zone de test, dans une deuxième forme, dans un récipient présentant un solvant; et
fc) analyse du solvant chargé de traces dans un analyseur;
fd) le résultat de l'analyse montrant si le procédé de nettoyage était couronné de succès ou si des résidus du liquide de test contaminé au-dessus de la tolérance sont restés sur la zone de test, de telle sorte que le procédé de nettoyage doit être répété ou intensifié dès le début pour atteindre un degré de pureté acceptable.

2. Procédé selon la revendication 1, **caractérisé en ce qu'**après l'application de la quantité définie de liquide de test sur la zone de texte et avant la réalisation du procédé de nettoyage standardisé de la zone de test pourvue du liquide de test, l'intensité et éventuellement aussi le type d'impureté présente sur la zone de test sont déterminés et consignés.

3. Procédé selon au moins l'une des revendications 1 et 2, **caractérisé en ce que** la zone de test, dans la première forme, est déterminée sur la surface:
a) d'une installation, par exemple d'une enceinte destinée à des fins de production ou de recherche de produits pharmaceutiques ou biotiques uniques ou en technique médicale; ou
b) d'une partie fonctionnelle utilisable dans l'installation.

4. Procédé selon au moins l'une des revendications 1 et 2, **caractérisé en ce que**, dans la deuxième forme alternative, une pièce de matériau séparée sous forme d'une plaquette indicatrice sert de zone de test, qui est identique en ce qui concerne sa caractéristique matérielle au matériau utilisé dans la construction de l'installation ou pour la pièce fonctionnelle ou du moins acceptable en tant que matériau de référence.

5. Procédé selon la revendication 4, **caractérisé en ce qu'**avant l'application du liquide de test sur la plaquette indicatrice choisie comme zone de test au moyen de l'appareil de revêtement, un dispositif de positionnement sert à l'orientation exacte de la plaquette indicatrice sous la tête d'impression de l'appareil de revêtement.

6. Procédé selon au moins l'une des revendications 1 à 5, **caractérisé en ce que** l'appareil de revêtement peut être réglé à différentes quantités de revêtement et différents motifs de revêtement.

7. Procédé selon au moins l'une des revendications 1 à 6, **caractérisé en ce que** l'appareil de revêtement peut être réglé à des zones de test de différentes dimensions.

8. Procédé selon au moins l'une des revendications 1 à 7, **caractérisé en ce que** l'appareil de revêtement est conçu comme un appareil à main.

9. Procédé selon au moins l'une des revendications 1 à 8, **caractérisé en ce que** la substance de contamination à ajouter au liquide de test est constituée de particules et/ou de produits de solvatation.

10. Procédé selon au moins l'une des revendications 1 à 9, **caractérisé en ce que** le liquide de test est constitué par:
a) un solvant neutre; et
b) des substances chimiques et/ou microbiologiques contenues dans le solvant, telles que des virus, des champignons, des spores, des bactéries, des protéines ou des fragments cellulaires, en une concentration définie pour la simulation d'impuretés sur la zone de test.

11. Procédé selon la revendication 10, **caractérisé en ce que** le liquide de test est constitué par:
a) un solvant neutre; et
b) des substances chimiques contenues dans le solvant et/ou un mélange à deux composants ou plus de substances microbiologiques du groupe constitué par les virus, les champignons, les spores, les bactéries, les protéines et les fragments cellulaires, en une concentration définie pour la simulation d'impuretés sur la zone de test.
